# EUROPEAN PATENT APPLICATION

(11) **EP 4 059 341 A1**
(43) Date of publication of application: **21.09.2022**
(21) Application number: 20886655.8
(22) Date of filing: 12.11.2020
(51) Int. Cl.: A01H 1/00, C12N 15/09, C12N 15/87

(54) **METHOD FOR INTRODUCING GENOME EDITING ENZYME INTO PLANT CELL USING PLASMA**

(30) Priority: 14.11.2019 JP 2019206239
(71) Applicant: NATIONAL AGRICULTURE AND FOOD RESEARCH ORGANIZATION, Tsukuba-shi, Ibaraki 305-8517 (JP)
(72) Inventor: MITSUHARA, Ichiro, Tsukuba-shi, Ibaraki 305-8634 (JP); YANAGAWA, Yuki, Tsukuba-shi, Ibaraki 305-8634 (JP); TOKI, Seiichi, Tsukuba-shi, Ibaraki 305-8634 (JP); IWAKAMI, Masaki, Tsukuba-shi, Ibaraki 305-8634 (JP); HIROSE, Sakiko, Tsukuba-shi, Ibaraki 305-8634 (JP); KATOH, Etsuko, Tsukuba-shi, Ibaraki 305-8518 (JP); OKINO, Akitoshi, Tokyo 152-8550 (JP); SUENAGA, Yuma, Tokyo 152-8550 (JP); MORIYA, Shohei, Tokyo 152-8550 (JP); IIJIMA, Yusuke, Tokyo 152-8550 (JP)
(74) Representative: J A Kemp LLP
(86) International application number: PCT/JP2020/042250
(87) International publication number: WO 2021/095802

(57) **Abstract**

A method for introducing a genome editing enzyme into a plant cell includes: treating the cell with plasma; and then bringing the cell into contact with the genome editing enzyme in the presence of a di- or higher-valent metal cation.

## Description

### Technical Field]

The present invention relates to a method for introducing a genome editing enzyme into a plant cell using plasma to cause genome editing.

### [Background Art]

The genome editing technique is a technique for creating a new cell or variety by introducing a mutation into a target site of a particular gene to modify the activity of a protein encoded thereon (for example, a substitution from an active form to an inactive form or a substitution from an inactive form to an active form) . This technique is capable of producing a variety or line in which a mutation is simply introduced into an endogenous gene and a foreign gene is not carried, and is different from the conventional gene recombination technique in that point.

Genome editing techniques such as ZFN, TALENs, and CRISPR-Cas systems have been developed so far and are attracting attention as useful tools for gene modification. For example, in the CRISPR-Cas system, a Cas protein forms a complex with a guide RNA, and this complex binds to a target site on a genome having a sequence complementary to the guide RNA, thereby cleaving both two strands of DNA. When this DNA double-strand break is repaired by non-homologous end joining, nucleotide insertion or deletion (insertion/deletion: indel) occurs, and gene knockout due to a frameshift or the like can be made. On the other hand, in the case where donor DNA to serve as a repair template is introduced from outside the cell, gene knockin can be made by homologous recombination between the genome and the donor DNA. In this gene knockin, it is possible not only to insert DNA but also to replace or delete one to several nucleotides.

When a genome editing enzyme such as a Cas protein is used for breeding crops, the mainstream method for plants is to introduce the enzyme gene by a gene recombination technique such as the Agrobacterium method (NPL 1). However, in the Agrobacterium method, the genome editing enzyme gene is integrated into the genomic DNA of the target plant, and it is necessary to remove the unnecessary enzyme gene after modifying the target gene of the plant. In this case, the genome editing enzyme gene can be removed from reproducible plants, but there are many crops such as vegetatively propagating plants and woody plants in which the unnecessary gene removal by reproduction is practically impossible.

To address this, the development of a technique for plants to directly introduce a genome editing enzyme as a protein into cells and perform genome editing without integrating the gene into the genome has been in progress, but applicable plant species are limited due to the necessity of making protoplasts or the like (NPLs 2 and 3). Similarly, there is a report that a target mutation was successful by directly introducing RNP (CRISPR/Cas9 protein RNA complex) into a maize embryo with a particle gun (NPL 4). However, in the method using a particle gun, even usual gene recombination only achieves regeneration of limited plants and the genome editing is considered to face much greater difficulty. There is also an attempt to perform virus-mediated genome editing of plants (NPL 5). However, due to a constraint on the length of a foreign gene that can be introduced into the virus, there has not been known any example in which genome editing by virus-mediated introduction of a genome editing enzyme was successful. In addition, when a recombinant virus is used, there are obstacles such as difficulty in demonstrating the absence of the virus in plants after genome editing. In addition to this, a method using a cell-penetrating peptide (NPL 6) and the like are known as a method for introducing a protein into plant cells, but there is no report on its application to plant genome editing.

Meanwhile, regarding the introduction of a substance such as a protein into a plant, the present inventors previously clarified that use of plasma makes it possible to easily and highly efficiently introduce a substance into a plant without causing a damage regardless of the types of the plant and tissue of the derivation (PTL 1).

### [Citation List]

### [Patent Literature]

[PTL 1] International Publication No. WO2018/016217

### [Non Patent Literature]

[NPL 1] Endo et al., Methods in Molecular Biology Volume 1469, pp. 123-135 (2016)
[NPL 2] Woo et al., Nature Biotech. 33: 1162-1164 (2016)
[NPL 3] Subburaj et al., Plant Cell Rep. 35: 1535-1544 (2016)
[NPL 4] Svitashev et al., Nature Communication 7: 13274 (2016)
[NPL 5] Ali et al., Molecular Plant 8: 1288-1291 (2015)
[NPL 6] Ng et al., Plos One 10: 1371 (2016)

### [Summary of Invention]

### [Technical Problem]

The present invention aims to provide a method for enabling genome editing with a genome editing enzyme by introducing the enzyme into a plant cell using plasma.

### [Solution to Problem]

The present inventors previously clarified that use of plasma makes it possible to easily and highly efficiently introduce a substance into a plant without causing a damage regardless of the types of the plant and tissue of the derivation (PTL 1). In particular, the present inventors clarified that a protein (sGFP-CyaA fusion protein) or DNA (plasmid DNA) can be introduced into plant cells by treating the cells with plasma and then bringing the substance into contact with the cell (PTL 1).

This time, the present inventors attempted to cause genome editing by introducing a CRISPR-Cas system (Cas protein and guide RNA), which is a type of genome editing system, into plant cells by the above introduction method using plasma.

However, the previous method had difficulty causing the genome editing using the complex of the Cas protein and the RNA unlike the foregoing protein or DNA.

To address this, the present inventors conducted intensive studies to cause genome editing by introducing the complex of the Cas protein and the RNA using plasma. As a result, the present inventors found that, when plant cells are treated with plasma and then the Cas protein and the guide RNA are brought into contact with the cells in the presence of a di- or higher-valent metal cation, the substance is introduced into the plant cells and genome editing can be achieved efficiently, and completed the present invention.

Specifically, the present invention relates to a method for introducing a genome editing enzyme such as a Cas protein into a plant cell using plasma, and more specifically provides the followings.
<1> A method for introducing a genome editing enzyme into a plant cell, comprising:
   treating the cell with plasma; and
   then bringing the cell into contact with the genome editing enzyme in the presence of a di- or higher-valent metal cation.
<2> A method for introducing a Cas protein and a guide RNA into a plant cell, comprising:
   treating the cell with plasma; and
   then bringing the cell into contact with the Cas protein and the guide RNA in the presence of a di- or higher-valent metal cation.
<3> The method according to <2>, wherein the Cas protein is a Cas9 protein.
<4> The method according to any one of <1> to <3>, wherein the di- or higher-valent metal cation is at least one divalent metal cation selected from the group consisting of Mg²⁺, Mn²⁺, Zn²⁺, Ca²⁺, and Ba²⁺.
<5> The method according to any one of <1> to <4>, wherein the plasma is normal temperature atmospheric pressure plasma.
<6> The method according to any one of <1> to <5>, wherein the plasma is at least one plasma selected from the group consisting of carbon dioxide plasma and nitrogen plasma.

### [Advantageous Effects of Invention]

According to the present invention, it is possible to cause genome editing by introducing a genome editing enzyme into a plant cell with a plasma treatment. In addition, as shown in Examples to be described later, the present invention makes it possible to cause genome editing by easily introducing a genome editing enzyme into a plant cell without causing a damage regardless of the types of the plant and tissue from which the plant cell is derived.

### [Brief Description of Drawings]

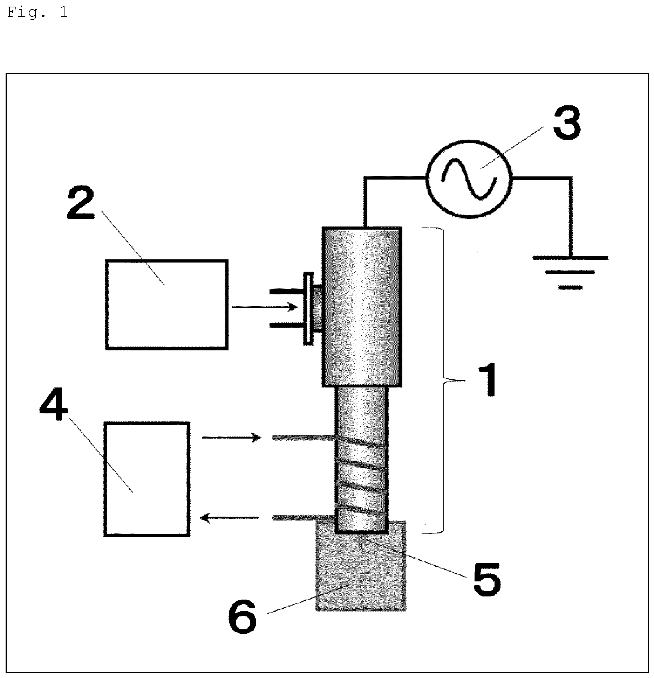

[Fig. 1] Fig. 1 is a schematic diagram illustrating an embodiment of a plasma treatment according to the present invention used in Examples. Specifically, Fig. 1 is a diagram showing that a flow of a plasma generation gas is fed into a plasma generator 1 from a gas supplier 2 while the gas is simultaneously cooled by a gas cooler 4, and thereafter a power supplier 3 applies a voltage to an internal electrode in the plasma generator to irradiate a sample 6 (plant cells) with plasma 5.

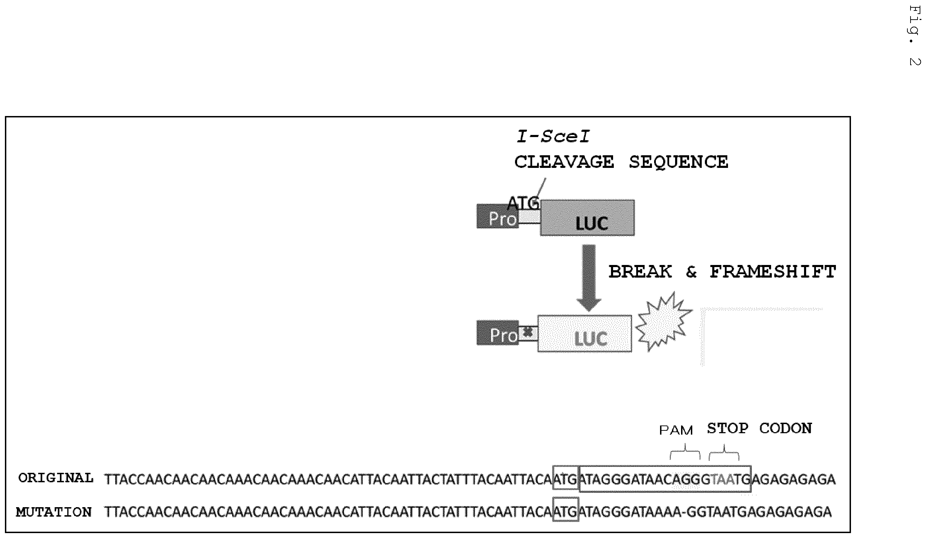

[Fig. 2] Fig. 2 is a diagram illustrating an outline of a reporter gene (L-(I-SceI)-UC) system used in Examples. The "original" and "mutation" sequences in Fig. 2 are set forth in SEQ ID NOs: 6 and 7, respectively.

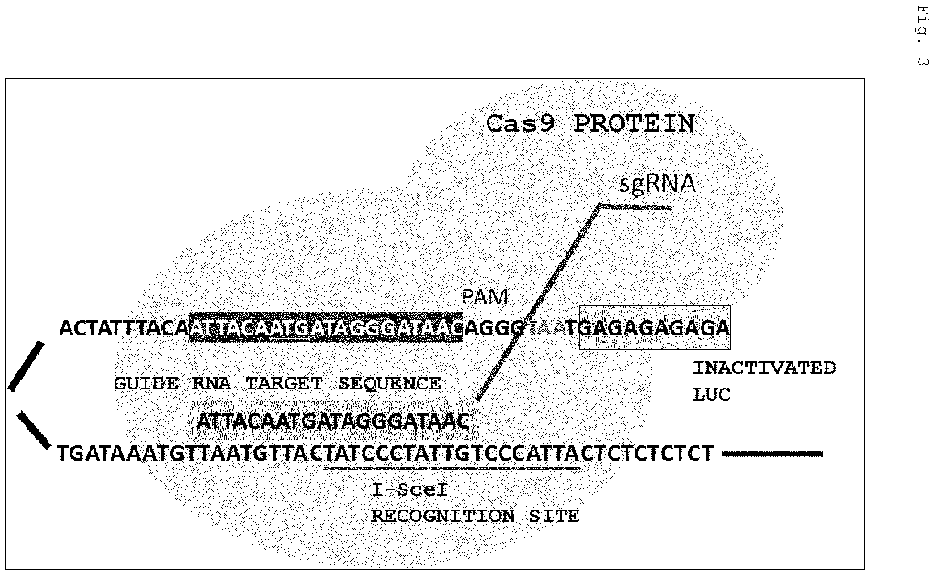

[Fig. 3] Fig. 3 is a schematic diagram illustrating a correspondence relationship between a reporter gene L-(I-SceI)-UC and a guide RNA (sgRNA) that targets it. A part of the L-(I-SceI)-UC sequence and a sequence complementary to it in Fig. 3 are set forth in SEQ ID NOs: 8 and 9, respectively. The guide RNA target sequence is set forth in SEQ ID NO: 10.

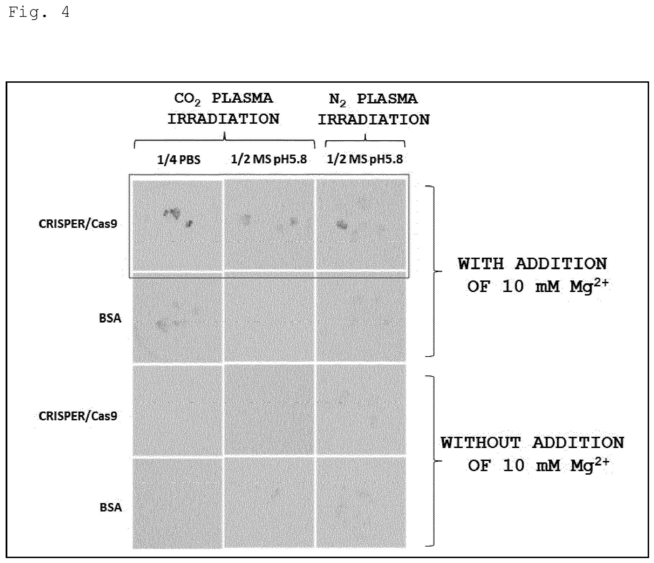

[Fig. 4] Fig. 4 presents photographs demonstrating results obtained by irradiating, with plasma, rice calluses to which the reporter gene L-(I-SceI)-UC was introduced, thereafter bringing the rice calluses into contact with a substance, and detecting the expression of the reporter gene (luciferin gene). In Fig. 4, the contacted substance (CRISPR/Cas9 (a complex of a Cas9 protein and a sgRNA) or BSA) is shown on the left side, the type of plasma for irradiation (CO₂ plasma or N₂ plasma) and the type of the medium (1/4 PBS or 1/2 MS (pH 5.8)) used for the contact are shown on the upper side, and whether Mg²⁺ was added to the buffer solution or the medium is shown on the right side.

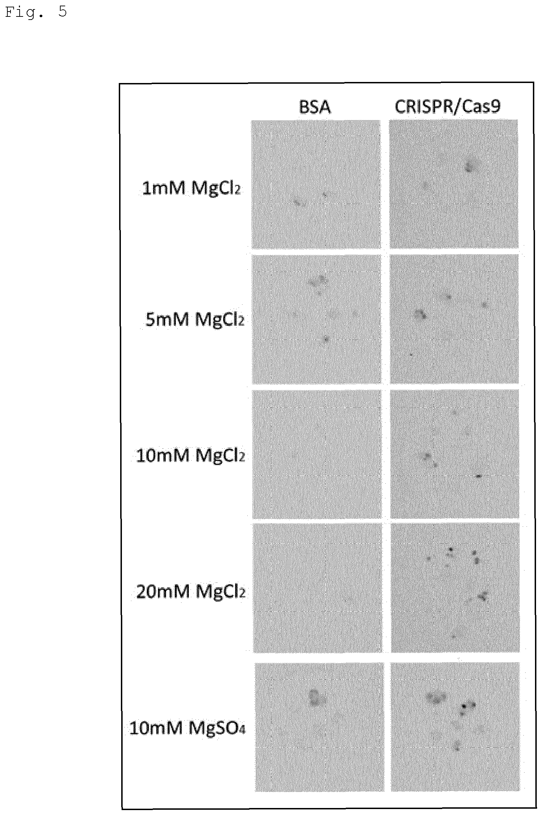

[Fig. 5] Fig. 5 presents photographs demonstrating results obtained by irradiating, with plasma, rice calluses to which the reporter gene L-(I-SceI)-UC was introduced, thereafter bringing the rice calluses into contact with a substance, and detecting the expression of the reporter gene (luciferin gene). In Fig. 5, the contacted substance (CRISPR/Cas9 (a complex of a Cas9 protein and a sgRNA) or BSA) is shown on the upper side, and the type and concentration of an inorganic salt added to a 1/2 MS (pH 5.8) medium used for the contact are shown on the left side.

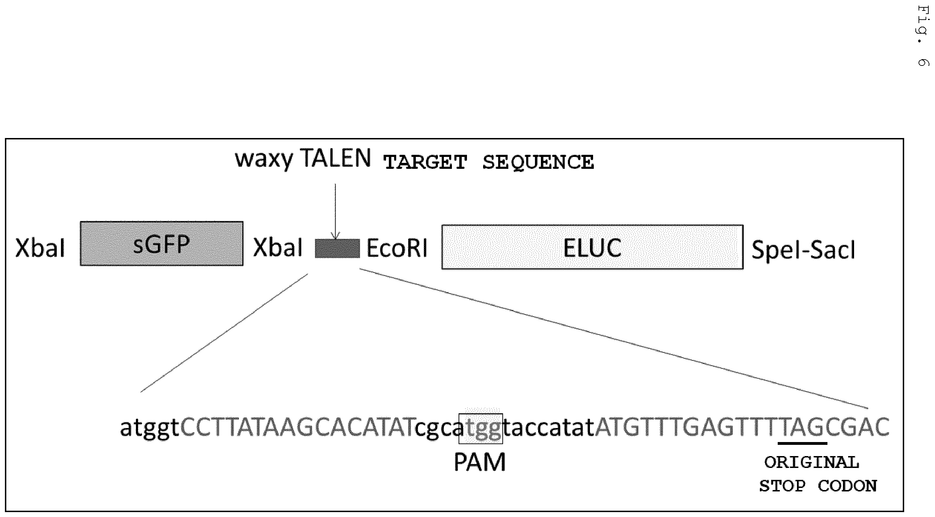

[Fig. 6] Fig. 6 is a diagram illustrating an outline of a reporter gene system pBI121-sGFP-wTALEN-ELUC. A waxy TALEN target sequence in Fig. 6 is set forth in SEQ ID NO: 12.

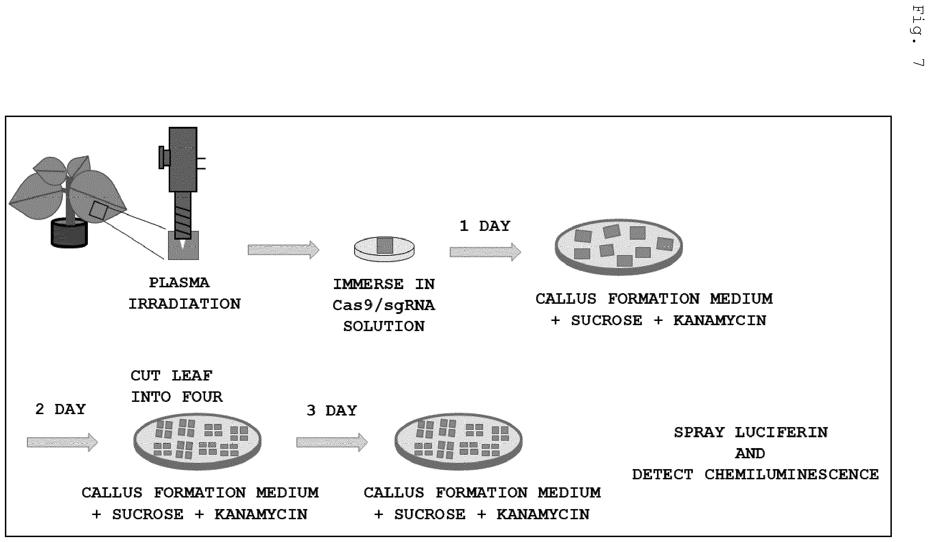

[Fig. 7] Fig. 7 is a schematic diagram illustrating a scheme including: irradiating, with plasma, a tobacco leaf to which the reporter gene system pBI121-sGFP-wTALEN-ELUC has been introduced; then bringing the tobacco leaf into contact with CRISPR/Cas9 (a complex of a Cas9 protein and a sgRNA) followed by culturing on a callus formation medium, and then detecting the expression of the reporter gene (luciferin gene).

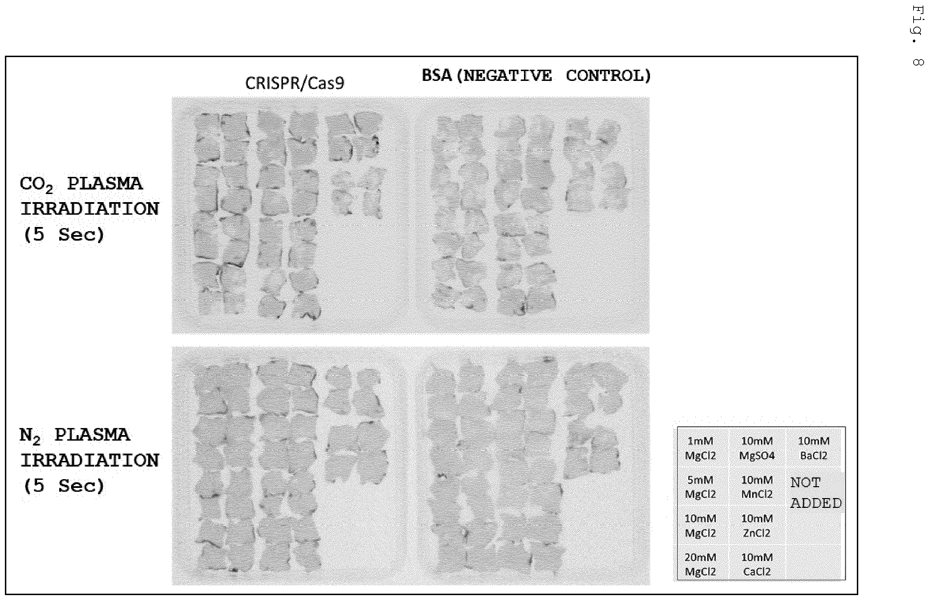

[Fig. 8] Fig. 8 presents photographs demonstrating results of detecting the expression of the reporter gene in accordance with the scheme illustrated in Fig. 7. In Fig. 8, the contacted substance (CRISPR/Cas9 (a complex of a Cas9 protein and a sgRNA) or BSA as a negative control) is shown on the upper side, and the type of plasma for irradiation (CO₂ plasma or N₂ plasma) is shown on the left side. In a small frame on the right lower side, the type and concentration of an inorganic salt added to a 1/2 MS (pH 5.8) medium used to bring each leaf into contact with the substance are shown.

### [Description of Embodiments]

### (Method for Introducing Substance into Plant Cells)

As shown in Examples to be described later, when plant cells are treated with plasma and then is brought into contact with a Cas protein and a guide RNA in the presence of di- or higher-valent metal cation, the substance can be introduced into the plant cells and thereby genome editing can be caused.

Therefore, the present invention provides a method for introducing a genome editing enzyme such as a Cas protein into plant cells, the method including treating the cell with plasma and then bringing the cell into contact with the genome editing enzyme in the presence of a di- or higher-valent metal cation.

In the present invention, the "genome editing enzyme" means an artificial restriction enzyme capable of causing a modification at a certain site on genomic DNA, and examples thereof include Cas, TALEN, ZFN, and PPR.

### (Cas)

In the present invention, the "Cas protein" is a CRISPER (clustered regularly interspaced short palindromic repeat)-related enzyme (nuclease), and may be a class 1 CRISPER-related enzyme (for example, type I such as Cas3, type IV, or type III such as Cas10) or a class 2 CRISPR-related enzyme (for example, type II such as Cas9, type V such as Cas12a (Cpf1), Cas12b (C2c1), Cas12e (CasX) and Cas14, or type VI such as Cas13), but is preferably the class 2 CRISPER-related enzyme, more preferably the type II CRISPER-related enzyme, and further preferably Cas9. Examples of the "Cas9 protein" include Streptococcus pyogenes (S. pyogenes) Cas9, Streptococcus pneumoniae (S. pneumoniae) Cas9, and S. thermophilus Cas9.

The typical amino acid sequence and base sequence of Cas protein are registered in a database open to the public, for example, GenBank (http://www.ncbi.nlm.nih.gov), and these can be used in the present invention. For example, the typical amino acid sequence and base sequence of the Cas9 protein derived from Streptococcus pyogenes are the sequences set forth in NCBI Reference Sequence: NP_269215 and NC_002737, respectively.

Further, the "Cas9 protein" according to the present invention is not limited to the protein comprising the above-mentioned typical amino acid sequence, but may be a homolog, a variant, or partial peptide thereof as long as it has the activity of forming a complex with the guide RNA and cleaving the target DNA.

Examples of the homolog include a protein comprising an amino acid sequence having an identity of 85% or more, preferably 90% or more, and more preferably 95% or more (for example, 96% or more, 97% or more, 98% or more, or 99% or more) with the amino acid sequence of a Cas protein of interest (for example, the amino acid sequence set forth in NP_269215). The sequence identity can be evaluated as a numeric value calculated by using BLAST or the like (for example, default or initially-set parameters).

Then, the valiant includes a protein comprising an amino acid sequence in which one or more amino acids are replaced, deleted, added, or inserted in the amino acid sequence of a natural Cas9 protein (for example, the amino acid sequence set forth in NP_269215) and which has a endonuclease activity. Here, "more amino acids" means, for example, 2 to 150 amino acids, preferably 2 to 100 amino acids, and more preferably 2 to 50 amino acids (for example, 2 to 30, 2 to 10, 2 to 5, 2 to 2, or 2 amino acids).

An example of the valiant is a Cas protein in which a PAM recognition specificity is modified by introducing a mutation into a specific amino acid residue. The technique for modifying the PAM recognition specificity in the Cas protein is known (Benjamin, P. et al., Nature 523, 481-485 (2015), and Hirano, S. et al., Molecular Cell 61, 886-894 (2016)).

To the Cas protein according to the present invention, a nuclear localization signal is preferably added. This promotes localization to the nucleus in the cell, resulting in efficient DNA editing.

Further, the Cas protein according to the present invention can be expressed as a recombinant protein by those skilled in the art by mounting the DNA encoding the Cas protein on an appropriate vector and introducing it into a host cell such as Escherichia coli, an animal cell, an insect cell, or a plant cell, or a cell-free protein synthesis system (for example, a reticulocyte extract or a wheat germ extract) . Then, the recombinant protein expressed in the host cell can be purified by a known peptide purification method. For example, the purification can be done by chromatography (for example, ion exchange affinity chromatography, size-exclusion column chromatography, or affinity chromatography using an antibody that specifically recognizes the Cas protein according to the present invention), centrifugation, or the like.

As the known method for purifying the recombinant protein expressed in the host cell, there is a purification method including: synthesizing the Cas protein in the form where a functional protein such as His-tag protein or glutathione-S-transferase protein is fused; and binding the Cas protein to a metal chelating resin, a GST affinitive resin, or the like (Smith, M. C. et al., J. Biol. Chem. 263, 7211-7215 (1988)). Also, for example, with TEV protease, thrombin, blood coagulation factor Xa, or the like, a recognition sequence thereof inserted between the functional protein and the Cas protein may be cleaved, so that only the Cas protein can be separated and purified.

The Cas protein according to the present invention can be prepared by chemical synthesis based on the amino acid sequence using a commercially available polypeptide synthesizer.

### (Guide RNA)

The Cas protein forming a complex with the guide RNA is thus induced into a target region, where the endonuclease activity of the Cas protein cleaves a target site, resulting in genome editing. Then, as shown in Examples to be described later, the present invention makes it possible to introduce not only the Cas protein but also the guide RNA into the plant cells. Thus, the substance to be introduced into plant cells in the present invention may include not only the Cas protein but also the guide RNA.

The "guide RNA" according to the present invention is a RNA containing a base sequence that interacts with the Cas protein (hereinafter also referred to as a "Cas interactive base sequence") and a base sequence complementary to the base sequence in a target region (hereinafter referred to as a "targeting base sequence").

The guide RNA of the CRISPR-Cas system according to the present invention may be a single-molecule guide RNA (sgRNA) containing crRNA and tracrRNA, or a double-molecule guide RNA containing a crRNA fragment and a tracrRNA fragment.

The targeting base sequence in the crRNA is a base sequence having usually 12 to 50 bases, preferably 17 to 30 bases, and more preferably 17 to 25 bases and is selected so as to target a region adjacent to a PAM (proto-spacer adjacent motif) sequence.

The crRNA also contains a base sequence capable of interacting (hybridizing) with the tracrRNA on the 3' side. On the other hand, the tracrRNA contains a base sequence capable of interacting (hybridizing) with the base sequence in a part of the crRNA on the 5' side. The double-stranded RNA formed by the interaction between these base sequences interacts with the Cas protein.

In the CRISPR-Cas system to be introduced into a plant cell, the guide RNA may be in the form of RNA, in the form of DNA encoding the RNA, or in the form of a vector to express the DNA.

When the form of RNA is employed, the guide RNA can be prepared by chemical synthesis based on the base sequence by using a commercially available polynucleotide synthesizer. Then, as shown in Examples to be described later, the guide RNA can be also prepared by an *in vitro* transcription system.

When the form of an expression vector is employed, the guide RNA contains one or more regulatory elements that operably binds to DNA to be expressed. Here, "operably (operable) binds" means that the above DNA expressively binds to the regulatory element. As the "regulatory elements", there are a promoter, an enhancer, an internal ribosome entry site (IRES), and other expression control elements (for example, transcription termination signal, and for example, polyadenylation signal and polyU sequence). The expression vector is preferably a vector capable of stably expressing an encoded protein without being integrated into the host genome.

### (TALEN)

According to the present invention, the "TALEN protein" is an artificial nuclease (TALEN) containing a DNA-binding domain of a transcriptional activator-like (TAL) effector in addition to a DNA-cleavage domain (for example, a FokI domain) (for example, US Patent Nos. 8470973 and 8586363). The TALEN introduced into a cell binds to the target site via the DNA-binding domain, and cleaves the DNA at that site. The DNA-binding domain to bind to the target site can be designed according to a known scheme (for example, Zhang, Feng et al., (2011) Nature Biotechnology 29 (2)).

### (ZFN)

According to the present invention, the "ZFN protein" is an artificial nuclease (ZFN) containing a nucleic acid-cleavage domain conjugated to a DNA-binding domain containing a zinc finger array (for example, US Patents Nos. 6265196 and 8524500 and EP Patent No. 1720995). The ZFN protein introduced into a cell binds to the target site via the DNA-binding domain, and cleaves the DNA at that site. The DNA-binding domain to bind to the target site can be designed according to a known scheme.

### (PPR)

According to the present invention, the "PPR protein" is an artificial nuclease containing a nucleic acid cleavage domain conjugated to a nucleic acid-binding domain containing a helix-loop-helix structure (PPR; pentatricopeptide repeat) (Nakamura et al., Plant Cell Physiol 53: 1171-1179 (2012)). The PPR protein introduced into a cell binds to a target site via the PPR motif, and cleaves the DNA at that site. Since correspondence relationships between various PPR motifs and bases recognized by the respective motifs are known, a nucleic acid-binding domain can be designed depending on a target site.

Here, these proteins TALEN, ZFN, and PPR can be prepared by those skilled in the art by using known methods as is the case with the Cas protein.

### (Plant Cell)

In the present invention, the "plant" is not particularly limited, and examples thereof include angiosperms encompassing dicotyledonous plants (tobacco, Arabidopsis thaliana, and the like) and monocotyledonous plants (rice and the like), gymnosperms, bryophytes, fern plants, herbaceous plants, and woody plants.

The "plant cell" in the present invention is not particularly limited and plant cells present in any tissue or plant cells derived from any tissue can be targeted. Examples of such tissues include leaves, roots, root ends, anthers, flowers, seeds, pods, stems, shoot apexes, embryos, and pollens. The method of the present invention can also target artificially treated plant cells (for example, calluses or suspension cultured cells). Further, the plant cell according to the present invention may be a cell having a cell wall, or may be a cell further having a cuticle layer outside the cell wall.

### (Plasma Treatment)

In the present invention, "plasma" includes a charged particle group in which molecules constituting a gas are divided into positive (positive ions) and negative (electrons) by ionization, and means a group of particles (ionized gas) which is almost electrically neutral as a whole. The "plasma" for treating plant cells is not particularly limited, and may be generated under atmospheric pressure (atmospheric pressure plasma) or may also be generated under pressure lower than the atmospheric pressure (low pressure plasma). However, the atmospheric pressure plasma is preferable from the viewpoints that the atmospheric pressure plasma does not require a vacuum system for its generation and is close to the living environment of plants. Note that in the present invention, the atmospheric pressure does not necessarily have to be strictly 1013 hPa, but may be in a range (500 to 2030 hPa) around the above pressure.

A method for generating atmospheric pressure plasma is not particularly limited, and those skilled in the art can appropriately perform the generation using a known method. Examples of the known method include dielectric barrier discharge, inductively coupled plasma discharge (ICP), capacitively coupled plasma discharge (CCP), hollow cathode discharge, corona discharge, streamer discharge, glow discharge, and arc discharge. Among these, the glow discharge and the hollow cathode discharge are preferable from the viewpoints that relatively high plasma, electron, and radical densities can be obtained and the gas temperature of the plasma can be kept low easily.

In addition, a current for causing discharge cannot be generally specified because it depends on the type of discharge, the size and shape of an apparatus for generating the discharge (thus plasma), the size and shape of the electrode to which a voltage is applied to generate the discharge, and so on, but may be a direct current or an alternating current.

The temperature of the "plasma" for treating the above plant cells is not particularly limited, and is usually -90 to 200°C, preferably -10 to 50°C, and more preferably 15 to 30°C (normal temperature). Such temperature control can be achieved by the method described in, for example, Oshita T, Kawano H, Takamatsu T, Miyahara H, Okino A (2015) "Temperature Controllable Atmospheric Plasma Source" IEEE Trans Sci43: 1987-1992, Japanese Unexamined Patent Application Publication No. 2010-061938, or the like. More specifically, according to the method, the gas to be used for generating the plasma to be described later is cooled to a low temperature (for example, -195°C) by a gas cooler using liquid nitrogen or the like; thereafter, the gas is heated to a desired temperature with a heater to generate the plasma, and further the gas temperature of the generated plasma is fed back to the heater, so that the temperature of the plasma can be controlled to a desired value by a step of 1°C.

The type of gas to which a voltage is applied in order to generate plasma is not particularly limited, but is preferably at least one gas selected from carbon dioxide, nitrogen, oxygen, hydrogen, and argon, and more preferably carbon dioxide and nitrogen from the viewpoint of introduction efficiency. Instead, as shown in Examples to be described later, a mixture gas composed of hydrogen and argon (preferably having a volume percentage of 0.01 to 50% hydrogen and 99.99 to 50% argon) or a mixture gas composed of nitrogen and oxygen (what is called air, preferably having a volume percentage of 90 to 70% nitrogen and 30 to 10% oxygen) may be also used.

Then, the rate of flow of the gas supplied to the plasma generator can be appropriately adjusted by those skilled in the art in consideration of the size, shape, and the like of the generator and moreover an aim to stabilize plasma generation while preventing the current of the plasma from blowing away a sample (plant cells), and an example of the rate of flow is 3 to 5 L/min.

The apparatus for generating plasma is not particularly limited, and a configuration as shown in Fig. 1 is presented as an example. More specifically, an apparatus for introducing a genome editing enzyme into a plant cell of the present invention preferably at least includes an apparatus (gas supplier 2) for supplying the generator with the gas to be used to generate plasma and an apparatus (power supplier 3) for supplying power to ionize the gas in addition to a plasma generator 1 capable of generating plasma, and more preferably further includes a gas cooler 4 for controlling the temperature of plasma and/or a table (mount table) where to mount a sample 6 (plant cells). Moreover, although not shown in Fig. 1, it is further preferable to provide, instead of the gas cooler 4, a gas cooling and gas heating system (gas temperature adjustment system) between the gas supplier 2 and the plasma generator 1. Furthermore, although not shown in Fig. 1, a heat insulating material may be provided instead of the gas cooler 4 in order to avoid the inflow of heat from the outside. Note that the plasma generator is not particularly limited, and any known apparatus may be appropriately used. For example, preferably used in the present invention are the apparatuses disclosed in Japanese Unexamined Patent Application Publication Nos. 2015-072913, 2014-212839, 2013-225421, 2013-094468, 2012-256501, 2008-041429, 2009-082796, and 2010-061938.

The treatment of plant cells with plasma generated as described above is usually accomplished by placing the cell below the plasma irradiation port followed by irradiation with plasma. In that case, the irradiation time is not particularly limited and can be appropriately adjusted depending on the types of plasma and the plant cells to be used, and the like. However, the irradiation time is preferably 0.01 to 3 minutes, more preferably 1 to 30 seconds, further preferably 1 to 10 seconds, and particularly preferably 2 to 5 seconds from the viewpoints that the efficiency of introduction of a genome editing enzyme is further enhanced while a damage on the plant cells is suppressed.

Furthermore, the distance from the plasma irradiation port to the plant cells is not particularly limited either, but it is desirable to appropriately adjust the distance from the plasma irradiation port because the plasma starts deactivation immediately after leaving the plasma generation section. The distance is preferably 1 to 100 mm and more preferably 5 to 20 mm. On the other hand, the plasma is required to be exhausted as a gas flow, and is also desired to uniformly irradiate the plant cells while inhibiting the plant cells from being blown away. In addition, those skilled in the art can appropriately adjust the distance so as to achieve both of the above-mentioned viewpoints, taking into consideration the types, sizes, and the like of the plasma apparatus, its gas flow, and plant cells to be used. For example, the distance from the plasma irradiation port to the plant cells is about 5 to 15 mm. From the same viewpoints, it is desirable to irradiate the plant cells directly with the plasma.

Instead, the plasma may be directly introduced into a liquid to cause bubbling, and then a plant may be immersed in the liquid or the liquid may be applied and sprayed onto the plant. This plasma treatment is useful in order to treat a large amount of plant cells uniformly. As the liquid, it is possible to use not only water or a culture liquid, but also a saline that interacts with the plasma and generates other active species. The temperature of the liquid is desirably about 5 to 20°C.

A start time of contact between the plant cells thus treated with plasma and the genome editing enzyme and the like to be introduced into the cells is not particularly limited, but is preferably between 0.01 to 30 minutes and more preferably 0.01 to 5 minutes after the treatment with plasma from the viewpoint that the introduction efficiency by plasma treatment can be further enhanced. Furthermore, a time duration of contact between the plant cells and the genome editing enzyme and the like is not particularly limited either, but is preferably 1 minute to 30 hours from the viewpoints of the introduction efficiency and the subsequent normal growth of the plant cells.

A method for bringing the plasma-treated plant cells into contact with the genome editing enzyme and the like is not particularly limited. The genome editing enzyme and the like themselves may be directly added to the plasma-contacted portions of the plant cells. Instead, the genome editing enzyme and the like may be added by being supported, added, mixed, or contained in a support for promoting the introduction. Examples of the support include phospholipid compositions such as liposomes, metals (such as gold and tungsten), particles or whiskers made of inorganic substances (such as silicon compounds), alginate beads, viral substances (for example, coat proteins), and cell penetrating peptides (CPP).

Moreover, the plant cells can be also brought into contact with the genome editing enzyme and the like by being added to a solution containing the genome editing enzyme and the like (or such as a mixture of a support and the genome editing enzyme and the like) or by being immersed in the solution. The solution is not particularly limited as long as the solution can keep the plant cells alive, and examples thereof include buffer solutions (for example, phosphate buffered saline (PBS), phosphate buffer solutions (NaPO₄ and KPO₄), HEPES buffer solutions, tris buffer solutions, MES buffer solutions, and citrate buffer solutions) and media (such as Murashige & Skoog (MS) medium). The compositions of the buffer solutions and the media are known, and the buffer solution or the medium may be diluted and used (for example, 1/4 PBS or 1/2 MS medium) depending on the types and the like of a plant and cells to be targeted in the present invention. The pH of the buffer solution and the medium can be also adjusted as appropriate depending on the types and the like of a plant and cells to be targeted, and is for example pH 5 to 8 and preferably pH 5.5 to 7.

The concentration of the genome editing enzyme in the solution can be adjusted as appropriate depending on a plant and cells to be targeted and the like, but is usually 1 to 100 µg/ml and preferably 10 to 50 µg/ml.

### (Di- or Higher-Valent Metal Cation)

In the present invention, the contact between the plant cells treated with the plasma as described above and a genome editing enzyme and the like for introducing the genome editing enzyme and the like into the cells is done in the presence of a di- or higher-valent metal cation.

Examples of the di- or higher-valent metal cation (multivalent metal cation) according to the present invention include divalent metal cations such as Mg²⁺, Mn²⁺, Zn²⁺, Ca²⁺, Ba²⁺, Cu²⁺, and Ni²⁺; trivalent metal cations such as Al³⁺; and a tetra- or higher-valent metal cations such as Cr⁶⁺. Among them, the divalent metal cations are preferable, and Mg²⁺, Mn²⁺, Zn²⁺, Ca²⁺, and Ba²⁺ are more preferable.

In the case where the plant cells were brought into contact with the substance in the solution, the concentration of the multivalent metal cation in the solution is usually 1 to 100 mM, preferably 2 to 50 mM, more preferably 3 to 30 mM, further preferably 5 to 25 mM, and even more preferably 10 to 20 mM. A counteranion for the multivalent metal cation is not particularly limited as shown in Examples to be described later, and examples thereof include Cl⁻, SO₄²⁻, and NO₃⁻.

### (Genome Editing)

In the present invention, a plant in which DNA is edited can be produced by regenerating the plant from plant cells into which a genome editing enzyme and the like are introduced. As a method for obtaining an individual by redifferentiating plant tissue by tissue culture, a method established in the field of the related art can be used ("Protocols for Plant Transformation" [Plant Edition] edited by Yutaka Tabei, Kagaku-Dojin Publishing Company, INC pp. 340-347 (2012)). Once a plant is obtained in this way, it is possible to obtain offspring from the plant by sexual reproduction or asexual reproduction. It is also possible to obtain breeding materials (for example, seeds, fruits, cut ears, strains, calluses, protoplasts, or the like) from the plant or its offspring or clones, and perform mass-production of the plant based on them.

### (Genome Editing Kit)

The present invention makes it possible to also provide a kit for use in the above method. The kit includes a solution containing a di- or higher-valent metal cation and may additionally include any of the follow substances as needed:
(1) Genome editing enzyme;
(2) Guide RNA or a vector to express the guide RNA in the case where the genome editing enzyme is a Cas protein; and
(3) Plasma generator.

Further, the kit of the present invention includes a manual instructing the above-mentioned introduction method and others.

### [Examples]

Hereinafter, the present invention will be described in more details based on Examples, but the present invention should not be limited to the following Examples. Examples were conducted by using the following method.

### <Cas9 Protein>

The Cas9 protein was prepared by using a pPURE vector (hereinafter also referred to as "pPURE-SpCas9") in which a Cas9 (SpCas9) gene derived from Streptococcus pyogenes was inserted. This vector was donated by Professor Osamu Nureki of the University of Tokyo, and encodes a SpCas9 protein (SpCas9-NLS-TEV-His6, SEQ ID NO: 1) in which SV40 nuclear localization signal (NLS), TEV protease recognition sequence, and histidine tag are fused to the C-terminal.

In the preparation of the above SpCas9 protein, more specifically, first, Escherichia coli Rosetta (DE3) (purchased from Novegen Ltd.) was transformed by introducing the pPURE-SpCas9. The obtained transformant was cultured by using an LB medium at 37°C. When OD600 was 0.6, 0.1 mM isopropyl β-D-1-thiogalactopyranoside was added to the medium to induce the expression of the SpCas9 protein. After that, the cells were cultured at 20°C for 12 hours, and then collected. The collected cells were suspended in 100 ml of a disruption buffer (50 mM Tris-HCl, pH 8.0, 500 mM NaCl, 25 mM imidazole) per 1 L of the cells and subjected to ultrasonic disruption treatment. The obtained disruption solution was subjected to centrifugation and the supernatant was applied to a HiTrap chelating HP column (purchased from GE Healthcare). After the column was washed with a disruption buffer, an elution buffer (50 mM Tris-HCl, pH 8.0, 500 mM NaCl, 500 mM imidazole) was passed therethrough to elute the SpCas9 protein. The crudely purified SpCas9 protein was applied to HiLoad 26/60 Superdex 200pg (purchased from GE Healthcare) equilibrated with a gel filtration buffer (20 mM Tris-HCl, pH 8.0, 300 mM NaCl). Peak fractions containing the SpCas9 protein were collected and concentrated by ultrafiltration using VivaSpin (registered trademark) Turbo 15, 50K (purchased from Sartorius) to a protein concentration of approximately 4 mg/ml and then were stored at -80°C.

### <sgRNA>

The sgRNAs (sgRNA for L-(I-SceI)-UC and the sgRNA for pBI121-sGFP-wTALEN-ELUC) were prepared by using a kit Guide-it^{™} sgRNA *In Vitro* Transcription and Screening System manufactured by Takara Bio Inc. according to the instructions in the attached manual.

Specifically, first, the following forward primers (I-SceI LUC Guide it and GFP-Wx-LUC Guide it) each containing a T7 promoter region, DNA encoding target sequence-specific crRNA, and a sequence homologous to a scaffold template were designed and chemically synthesized.
(I-SceI LUC Guide it) 
5'TGCGGCCTCTAATACGACTCACTATAGGGATTACAATGATAGGGATAACGTT

TTAGAGCTAGAAATAGCA3' (SEQ ID NO: 2)
(GFP-Wx-LUC Guide it)

5'TGCGGCCTCTAATACGACTCACTATAGGGCCTTATAAGCACATATCGCAGTT

TTAGAGCTAGAAATAGCA3' (SEQ ID NO: 3).

Next, by using the following scaffold template (DNA encoding tracrRNA, Guide-it scaffold template) as a template, a template DNA for *in vitro* transcription (DNA containing the T7 promoter region, the DNA encoding the target sequence-specific crRNA, and the DNA encoding tracrRNA) was produced by PCR using each of the above forward primers.
(Guide-it Scaffold Template)

5'GCACCGACTCGGTGCCACTTTTTCAAGTTGATAACGGACTAGCCTTATTTTA

ACTTGCTATTTCTAGCTCTAAAAC3' (SEQ ID NO: 4).

Then, each sgRNA was prepared using the corresponding one of the template DNAs and a ScriptMAX (registered trademark) Thermo T7 transcription kit manufactured by TOYOBO CO., LTD.

### <Cas9/sgRNA Complex>

The SpCas9 protein and the sgRNA were added to a 1x NEB3 buffer in a mass ratio of 5:1, followed by incubation on ice for 30 minutes to form a complex of them.

### (Plasma Treatment)

The plasma treatment was conducted in accordance with the methods described in Takamatsu T, Hirai H, Sasaki R, Miyahara H, Okino A (2013) "Surface Hydrophilization of Polyimide Films Using Atmospheric Damage-Free Multigas Plasma Jet Source" IEEE Trans. Plasma Sci 41: 119-125 and Oshita T, Kawano H, Takamatsu T, Miyahara H, Okino A (2015) "Temperature Controllable Atmospheric Plasma Source" IEEE Trans Sci 43: 1987-1992.

More specifically, as shown in Fig. 1, the apparatus main body of a plasma generator (Damage-Free Multigas Plasma Jet manufactured by Plasma Concept Tokyo Inc. (Damage-Free Plasma (Japanese registered trademark No. 5409073)) and Multigas Plasma (Japanese registered trademark No. 5432585)), product number: PCT-DFMJ02)) was grounded, and a certain high voltage was supplied to an internal high-voltage electrode of a plasma generation section from the apparatus main body. The certain high voltage was a modulated alternating voltage of 10 to 30 kHz with a maximum of 9 kV. When supplied with the electric power, the plasma generation section generated glow discharge and then generated stable atmospheric pressure plasma by receiving, as a gas species, carbon dioxide or nitrogen flowing in through a 1-mm hole at a rate of flow of 5 L/min.

The temperature of the plasma thus generated (the temperature at 5 mm away from the plasma irradiation port) was found to be 50°C or less as a result of thermocouple measurement. In order to generate plasma having a lower temperature (about 15 to 30°C), the gas was cooled by a gas cooling apparatus using liquid nitrogen.

Then, the irradiation port was set at a position 5 mm vertically above the plant tissue and the plant tissue was directly treated with the plasma for 5 seconds. After that, the following introduction medium or a phosphate buffered saline (1/4 PBS) containing the Cas9/sgRNA complex or a bovine serum albumin (BSA) was brought into contact with the plant tissue.

### <Cas9/sgRNA Introduction Solution>

A Cas9/sgRNA introduction medium was prepared by adding the Cas9/sgRNA or BSA at a protein content of 50 µg/ml to a 1/2 MS liquid medium [1/2 bag/L of Murashige and Skoog plant salt mixture (manufactured by Wako cooperation), pH 5.8] or the 1/4 PBS to each of which 1 to 20 mM MgCl₂, 10 mM MgSO₄, or another divalent cation (MnCl₂, ZnCl₂, CaCl₂, or BaCl₂) at 10 mM was added.

### <Rice Callus>

Agrobacterium transformed with a binary vector having an L-(I-SceI)-UC and NPTII expression construct (hereinafter also referred to as "pZK-L-I-SceI-UC", consisting of a DNA sequence set forth in SEQ ID NO: 5) was prepared. Then, based on the method described in Saika et al., Transgenic Res. (2012): 67-74, rice calluses were affected with the Agrobacterium, and transformed calluses were selected by using G418.

In L-(I-SceI)-UC, as illustrated in Fig. 2, the I-SceI recognition sequence is inserted immediately after the start codon of the luciferase gene, and the stop codon in-frame with the start codon is present in the recognition sequence. For this reason, this reporter gene is incapable of expressing the luciferase protein in general. On the other hand, in a case where the recognition sequence is cleaved by the Cas9/sgRNA (see Fig. 3) that recognizes the recognition sequence and is repaired (genome edited) so that a frameshift occurs, the luciferase protein can be expressed along with 1-base deletion, 2-base insertion, or the like.

### <Plasma Treatment and Genome Editing on Rice Callus>

The rice calluses to express the reporter gene system (L-(I-SceI)-UC) were irradiated with CO₂ plasma or N₂ plasma for 5 seconds in a clean bench. Then, the rice calluses after the plasma irradiation were immersed in the Cas9/sgRNA introduction solution, left for one day at room temperature, then transferred to a 12-well plate in which each well contained 2.5 ml of N6d gelrite medium, and cultured for 2 to 5 days in an artificial climate chamber at 28°C with 16 hours light and 8 hours darkness. The N6d gelrite medium was a N6d medium containing NAA 0.1 mg/L, sucrose 30 g/L, and gelrite 4 g/L [1x Chu (N69) medium salt mixture (manufacture by Wako Corporation) 1 bag/L, glycine 2 mg/L, nicotinic acid 0.5 mg/L, pyridoxine hydrochloride 0.5 mg/L, thiamine hydrochloride 1 mg/L, L-proline 2.878 g/L, casamino acid 0.3 g/L, pH 5.8].

### <Tobacco leaf>

Tobacco (Nicotiana tabacum cv. Samsun NN) was cultivated at 25°C in 16 hours light/8 hours dark cycles after the seeds thereof was sown in soil. Then, in accordance with the methods described in Mitsuhara et al., Plant Cell Physiol. (1996) 37: 49-59 and Horsch et al., Science (1985) 227: 1229-1231, the pBI121-sGFP-wTALEN-ELUC was introduced to the leaves (mature leaves) 4 to 8 weeks after the sowing, thereby producing a transformant.

In a reporter gene system psGFP-wTALEN-ELUC (consisting of a DNA sequence set forth in SEQ ID NO: 11) possessed by the pBI121-sGFP-wTALEN-ELUC, the green fluorescent protein (sGFP) and the luciferase gene (ELUC) are aligned across a spacer region, a fragment of a rice Waxy gene partially modified is inserted in the spacer region, and the coding region of the sGFP gene and the coding region of the ELUC gene are purposely aligned out of frame with each other. Therefore, since the stop codon appears in the spacer sequence part as illustrated in Fig. 6, the translation from mRNA transcribed from this reporter gene system stops at a position where the translation of the sGFP gene part is completed, and the luciferase protein is not expressed. On the other hand, in a case where the spacer region is cleaved by the Cas9/sgRNA that recognizes the spacer region and is repaired (genome edited) so that a frameshift occurs, the luciferase protein can be expressed along with 1-base deletion, 2-base insertion, or the like.

### <Plasma Treatment on Tobacco Leaf>

For plasma irradiation, tobacco leaves were surface-sterilized with 70% ethanol and 1% sodium hypochlorite, and then cut into square pieces of about 1.5 to 2 cm on a paper towel. Then, the square pieces were arranged on a 1/2 MS agar plate and left at room temperature for 1 day. These leaves were irradiated with CO₂ plasma or N₂ plasma for 5 seconds. Then, the tobacco leaves after the plasma irradiation were immersed in the Cas9/sgRNA introduction solution, left at room temperature for 1 day, and then arranged on a callus formation medium containing 30 g of sucrose and 50 µg/ml kanamycin [1x Murashige and Skoog (MS), 1x MS Vitamin (0.1 µg/ml thiamine hydrochloride, 0.5 µg/ml pyridoxine hydrochloride, 0.5 µg/ml nicotinamide, 2 µg/ml glycine, 100 µg/ml myo-inositol), 0.1 µg/ml α-naphthalene acetate, 1 µg/ml 6-benzylaminopurine, 8.5 g/L agar, pH 8.5] and left at 28°C for 2 days in 16 hours light/8 hours dark cycles. One leaf was cut into 4 pieces, which were arranged again on a callus formation medium containing 30 g of sucrose and lest at 28°C for 3 days in 16 hours light/8 hours dark cycles.

### <Judgment of Genome Editing>

Selection of genome-edited tissues was performed using luciferase activity as an index. Specifically, a sodium phosphate buffer (pH 7.0) containing 1 mM luciferin was sprayed on each tissue, and detection of the luciferase activity was performed by a chemiluminescence detection system (LAS-3000, manufactured by Fuji Photo Film Co., Ltd.).

### (Example 1) Introduction of Cas9/sgRNA Complex into Rice Callus with Plasma

The rice calluses to which the reporter gene illustrated in Fig. 2 was introduced were irradiated with plasma to introduce the complex of the Cas9 protein and the sgRNA illustrated in Fig. 3, and then detection of genome editing was attempted.

As a result, as illustrated in Fig. 4, the genome editing by introducing the Cas9/sgRNA complex in the conventional substance introduction method with plasma treatment (the method described in PTL 1) was not detected (see three photographs on the third row from the top in Fig. 4). On the other hand, as a result of bringing the rice calluses after the plasma irradiation into contact with the Cas9/sgRNA complex in the 1/4 PBS or 1/2 MS medium to which 10 mM Mg²⁺ was added, it was found that the genome editing efficiently occurred (see three photographs on the first row from the top in Fig. 4) .

In addition, as shown in Fig. 5, it was also found that even when the type of counterions (Cl⁻ or SO₄²⁻) for Mg²⁺ was changed, the genome editing was possible with addition of Mg²⁺ (1 to 20 mM) to the medium for contact with the Cas9/sgRNA complex. Moreover, there was a tendency that the higher the concentration of MgCl₂ added, the more noticeable the difference from the negative control.

Although not shown in the drawings, as a result of a viability assay with neutral red staining after the plasma irradiation and the subsequent immersion in the Cas9/sgRNA introduction solution, the survival of the rise calluses was observed regardless whether Mg²⁺ was added to the solution, which revealed that the genome editing was possible without causing a damage.

### (Example 2) Introduction of Cas9/sgRNA Complex into Tobacco Leaf with Plasma

The tobacco leaves to which the reporter gene illustrated in Fig. 6 was introduced were irradiated with the plasma, thus the complex of the Cas9 protein and the sgRNA was introduced to the tobacco leaves, and then detection of genome editing was attempted in a method illustrated in Fig. 7.

As shown in Fig. 8, the result also revealed that the addition of Mg²⁺ (1 to 20 mM) to the medium for contact with the Cas9/sgRNA complex enabled genome editing also in the tobacco leaves regardless the type of counterions for Mg²⁺.

Moreover, it was further revealed that even a divalent metal cation (Mn²⁺, Zn²⁺, Ca²⁺, or Ba²⁺) other than Mg²⁺ also enabled genome editing with the plasma treatment.

### [Industrial Applicability]

The present invention makes it possible to cause genome editing by introducing a genome editing enzyme such as a Cas protein into a plant cell with a plasma treatment as described above. In addition, the present invention makes it possible to easily introduce a genome editing enzyme into a plant cell without causing a damage regardless of the types of the plant and tissue from which the plant cell is derived, thereby causing genome editing.

Therefore, according to the method of the present invention, it is possible to efficiently produce new cells or variety in which a mutation is simply introduced into an endogenous gene through genome editing and a foreign gene is not carried. This is very useful in basic research because it is possible to analyze the function of the endogenous gene into which the mutation is introduced by checking a change in the phenotype of plant cells or the like. In addition, plant cells to which new functions are added by genome editing are also very useful in the fields of production and development of biomass, functional foodstuffs, pharmaceutical materials, and so on. Therefore, the present invention makes a great contribution also to various industrial applications.

### [Reference Signs List]

### [Reference Signs List]

- 1:: plasma generator
- 2:: gas supplier
- 3:: power supplier
- 4:: gas cooler
- 5:: plasma
- 6:: sample

## Claims

1. A method for introducing a genome editing enzyme into a plant cell, comprising:
treating the cell with plasma; and
then bringing the cell into contact with the genome editing enzyme in the presence of a di- or higher-valent metal cation.

2. A method for introducing a Cas protein and a guide RNA into a plant cell, comprising:
treating the cell with plasma; and
then bringing the cell into contact with the Cas protein and the guide RNA in the presence of a di- or higher-valent metal cation.

3. The method according to claim 2, wherein the Cas protein is a Cas9 protein.

4. The method according to any one of claims 1 to 3, wherein the di- or higher-valent metal cation is at least one divalent metal cation selected from the group consisting of Mg²⁺, Mn²⁺, Zn²⁺, Ca²⁺, and Ba²⁺.

5. The method according to any one of claims 1 to 4, wherein the plasma is normal temperature atmospheric pressure plasma.

6. The method according to any one of claims 1 to 5, wherein the plasma is at least one plasma selected from the group consisting of carbon dioxide plasma and nitrogen plasma.


SEQUENCE LISTING

<110>  NATIONAL AGRICULTURE AND FOOD RESEARCH ORGANIZATION

<120>  プラズマを用いてゲノム編集酵素を植物細胞内に導入する方法

<130>  IBPF20-544WO

<150>  JP2019-206239
<151>  2019-11-14

<160>  12

<170>  PatentIn version 3.5

<210>  1
<211>  1393
<212>  PRT
<213>  Artificial Sequence

<220>
<223>  SpCas9-NLS-TEV-His6

<400>  1

Met Asp Lys Lys Tyr Ser Ile Gly Leu Asp Ile Gly Thr Asn Ser Val
1               5                   10                  15

Gly Trp Ala Val Ile Thr Asp Glu Tyr Lys Val Pro Ser Lys Lys Phe
            20                  25                  30

Lys Val Leu Gly Asn Thr Asp Arg His Ser Ile Lys Lys Asn Leu Ile
        35                  40                  45

Gly Ala Leu Leu Phe Asp Ser Gly Glu Thr Ala Glu Ala Thr Arg Leu
    50                  55                  60

Lys Arg Thr Ala Arg Arg Arg Tyr Thr Arg Arg Lys Asn Arg Ile Cys
65                  70                  75                  80

Tyr Leu Gln Glu Ile Phe Ser Asn Glu Met Ala Lys Val Asp Asp Ser
                85                  90                  95

Phe Phe His Arg Leu Glu Glu Ser Phe Leu Val Glu Glu Asp Lys Lys
            100                 105                 110

His Glu Arg His Pro Ile Phe Gly Asn Ile Val Asp Glu Val Ala Tyr
        115                 120                 125

His Glu Lys Tyr Pro Thr Ile Tyr His Leu Arg Lys Lys Leu Val Asp

130                 135                 140

Ser Thr Asp Lys Ala Asp Leu Arg Leu Ile Tyr Leu Ala Leu Ala His
145                 150                 155                 160

Met Ile Lys Phe Arg Gly His Phe Leu Ile Glu Gly Asp Leu Asn Pro
                165                 170                 175

Asp Asn Ser Asp Val Asp Lys Leu Phe Ile Gln Leu Val Gln Thr Tyr
            180                 185                 190

Asn Gln Leu Phe Glu Glu Asn Pro Ile Asn Ala Ser Gly Val Asp Ala
        195                 200                 205

Lys Ala Ile Leu Ser Ala Arg Leu Ser Lys Ser Arg Arg Leu Glu Asn
    210                 215                 220

Leu Ile Ala Gln Leu Pro Gly Glu Lys Lys Asn Gly Leu Phe Gly Asn
225                 230                 235                 240

Leu Ile Ala Leu Ser Leu Gly Leu Thr Pro Asn Phe Lys Ser Asn Phe
                245                 250                 255

Asp Leu Ala Glu Asp Ala Lys Leu Gln Leu Ser Lys Asp Thr Tyr Asp
            260                 265                 270

Asp Asp Leu Asp Asn Leu Leu Ala Gln Ile Gly Asp Gln Tyr Ala Asp
        275                 280                 285

Leu Phe Leu Ala Ala Lys Asn Leu Ser Asp Ala Ile Leu Leu Ser Asp
    290                 295                 300

Ile Leu Arg Val Asn Thr Glu Ile Thr Lys Ala Pro Leu Ser Ala Ser
305                 310                 315                 320

Met Ile Lys Arg Tyr Asp Glu His His Gln Asp Leu Thr Leu Leu Lys
                325                 330                 335

Ala Leu Val Arg Gln Gln Leu Pro Glu Lys Tyr Lys Glu Ile Phe Phe
            340                 345                 350

Asp Gln Ser Lys Asn Gly Tyr Ala Gly Tyr Ile Asp Gly Gly Ala Ser
        355                 360                 365

Gln Glu Glu Phe Tyr Lys Phe Ile Lys Pro Ile Leu Glu Lys Met Asp
    370                 375                 380

Gly Thr Glu Glu Leu Leu Val Lys Leu Asn Arg Glu Asp Leu Leu Arg
385                 390                 395                 400

Lys Gln Arg Thr Phe Asp Asn Gly Ser Ile Pro His Gln Ile His Leu
                405                 410                 415

Gly Glu Leu His Ala Ile Leu Arg Arg Gln Glu Asp Phe Tyr Pro Phe
            420                 425                 430

Leu Lys Asp Asn Arg Glu Lys Ile Glu Lys Ile Leu Thr Phe Arg Ile
        435                 440                 445

Pro Tyr Tyr Val Gly Pro Leu Ala Arg Gly Asn Ser Arg Phe Ala Trp
    450                 455                 460

Met Thr Arg Lys Ser Glu Glu Thr Ile Thr Pro Trp Asn Phe Glu Glu
465                 470                 475                 480

Val Val Asp Lys Gly Ala Ser Ala Gln Ser Phe Ile Glu Arg Met Thr
                485                 490                 495

Asn Phe Asp Lys Asn Leu Pro Asn Glu Lys Val Leu Pro Lys His Ser
            500                 505                 510

Leu Leu Tyr Glu Tyr Phe Thr Val Tyr Asn Glu Leu Thr Lys Val Lys
        515                 520                 525

Tyr Val Thr Glu Gly Met Arg Lys Pro Ala Phe Leu Ser Gly Glu Gln
    530                 535                 540

Lys Lys Ala Ile Val Asp Leu Leu Phe Lys Thr Asn Arg Lys Val Thr
545                 550                 555                 560

Val Lys Gln Leu Lys Glu Asp Tyr Phe Lys Lys Ile Glu Cys Phe Asp
                565                 570                 575

Ser Val Glu Ile Ser Gly Val Glu Asp Arg Phe Asn Ala Ser Leu Gly
            580                 585                 590

Thr Tyr His Asp Leu Leu Lys Ile Ile Lys Asp Lys Asp Phe Leu Asp

595                 600                 605

Asn Glu Glu Asn Glu Asp Ile Leu Glu Asp Ile Val Leu Thr Leu Thr
    610                 615                 620

Leu Phe Glu Asp Arg Glu Met Ile Glu Glu Arg Leu Lys Thr Tyr Ala
625                 630                 635                 640

His Leu Phe Asp Asp Lys Val Met Lys Gln Leu Lys Arg Arg Arg Tyr
                645                 650                 655

Thr Gly Trp Gly Arg Leu Ser Arg Lys Leu Ile Asn Gly Ile Arg Asp
            660                 665                 670

Lys Gln Ser Gly Lys Thr Ile Leu Asp Phe Leu Lys Ser Asp Gly Phe
        675                 680                 685

Ala Asn Arg Asn Phe Met Gln Leu Ile His Asp Asp Ser Leu Thr Phe
    690                 695                 700

Lys Glu Asp Ile Gln Lys Ala Gln Val Ser Gly Gln Gly Asp Ser Leu
705                 710                 715                 720

His Glu His Ile Ala Asn Leu Ala Gly Ser Pro Ala Ile Lys Lys Gly
                725                 730                 735

Ile Leu Gln Thr Val Lys Val Val Asp Glu Leu Val Lys Val Met Gly
            740                 745                 750

Arg His Lys Pro Glu Asn Ile Val Ile Glu Met Ala Arg Glu Asn Gln
        755                 760                 765

Thr Thr Gln Lys Gly Gln Lys Asn Ser Arg Glu Arg Met Lys Arg Ile
    770                 775                 780

Glu Glu Gly Ile Lys Glu Leu Gly Ser Gln Ile Leu Lys Glu His Pro
785                 790                 795                 800

Val Glu Asn Thr Gln Leu Gln Asn Glu Lys Leu Tyr Leu Tyr Tyr Leu
                805                 810                 815

Gln Asn Gly Arg Asp Met Tyr Val Asp Gln Glu Leu Asp Ile Asn Arg
            820                 825                 830

Leu Ser Asp Tyr Asp Val Asp His Ile Val Pro Gln Ser Phe Leu Lys
        835                 840                 845

Asp Asp Ser Ile Asp Asn Lys Val Leu Thr Arg Ser Asp Lys Asn Arg
    850                 855                 860

Gly Lys Ser Asp Asn Val Pro Ser Glu Glu Val Val Lys Lys Met Lys
865                 870                 875                 880

Asn Tyr Trp Arg Gln Leu Leu Asn Ala Lys Leu Ile Thr Gln Arg Lys
                885                 890                 895

Phe Asp Asn Leu Thr Lys Ala Glu Arg Gly Gly Leu Ser Glu Leu Asp
            900                 905                 910

Lys Ala Gly Phe Ile Lys Arg Gln Leu Val Glu Thr Arg Gln Ile Thr
        915                 920                 925

Lys His Val Ala Gln Ile Leu Asp Ser Arg Met Asn Thr Lys Tyr Asp
    930                 935                 940

Glu Asn Asp Lys Leu Ile Arg Glu Val Lys Val Ile Thr Leu Lys Ser
945                 950                 955                 960

Lys Leu Val Ser Asp Phe Arg Lys Asp Phe Gln Phe Tyr Lys Val Arg
                965                 970                 975

Glu Ile Asn Asn Tyr His His Ala His Asp Ala Tyr Leu Asn Ala Val
            980                 985                 990

Val Gly Thr Ala Leu Ile Lys Lys Tyr Pro Lys Leu Glu Ser Glu Phe
        995                 1000                1005

Val Tyr Gly Asp Tyr Lys Val Tyr Asp Val Arg Lys Met Ile Ala
    1010                1015                1020

Lys Ser Glu Gln Glu Ile Gly Lys Ala Thr Ala Lys Tyr Phe Phe
    1025                1030                1035

Tyr Ser Asn Ile Met Asn Phe Phe Lys Thr Glu Ile Thr Leu Ala
    1040                1045                1050

Asn Gly Glu Ile Arg Lys Arg Pro Leu Ile Glu Thr Asn Gly Glu

1055                 1060                 1065

Thr Gly  Glu Ile Val Trp Asp  Lys Gly Arg Asp Phe  Ala Thr Val
    1070                 1075                 1080

Arg Lys  Val Leu Ser Met Pro  Gln Val Asn Ile Val  Lys Lys Thr
    1085                 1090                 1095

Glu Val  Gln Thr Gly Gly Phe  Ser Lys Glu Ser Ile  Leu Pro Lys
    1100                 1105                 1110

Arg Asn  Ser Asp Lys Leu Ile  Ala Arg Lys Lys Asp  Trp Asp Pro
    1115                 1120                 1125

Lys Lys  Tyr Gly Gly Phe Asp  Ser Pro Thr Val Ala  Tyr Ser Val
    1130                 1135                 1140

Leu Val  Val Ala Lys Val Glu  Lys Gly Lys Ser Lys  Lys Leu Lys
    1145                 1150                 1155

Ser Val  Lys Glu Leu Leu Gly  Ile Thr Ile Met Glu  Arg Ser Ser
    1160                 1165                 1170

Phe Glu  Lys Asn Pro Ile Asp  Phe Leu Glu Ala Lys  Gly Tyr Lys
    1175                 1180                 1185

Glu Val  Lys Lys Asp Leu Ile  Ile Lys Leu Pro Lys  Tyr Ser Leu
    1190                 1195                 1200

Phe Glu  Leu Glu Asn Gly Arg  Lys Arg Met Leu Ala  Ser Ala Gly
    1205                 1210                 1215

Glu Leu  Gln Lys Gly Asn Glu  Leu Ala Leu Pro Ser  Lys Tyr Val
    1220                 1225                 1230

Asn Phe  Leu Tyr Leu Ala Ser  His Tyr Glu Lys Leu  Lys Gly Ser
    1235                 1240                 1245

Pro Glu  Asp Asn Glu Gln Lys  Gln Leu Phe Val Glu  Gln His Lys
    1250                 1255                 1260

His Tyr  Leu Asp Glu Ile Ile  Glu Gln Ile Ser Glu  Phe Ser Lys
    1265                 1270                 1275

Arg Val Ile Leu Ala Asp Ala Asn Leu Asp Lys Val Leu Ser Ala
    1280                1285                1290

Tyr Asn Lys His Arg Asp Lys Pro Ile Arg Glu Gln Ala Glu Asn
    1295                1300                1305

Ile Ile His Leu Phe Thr Leu Thr Asn Leu Gly Ala Pro Ala Ala
    1310                1315                1320

Phe Lys Tyr Phe Asp Thr Thr Ile Asp Arg Lys Arg Tyr Thr Ser
    1325                1330                1335

Thr Lys Glu Val Leu Asp Ala Thr Leu Ile His Gln Ser Ile Thr
    1340                1345                1350

Gly Leu Tyr Glu Thr Arg Ile Asp Leu Ser Gln Leu Gly Gly Asp
    1355                1360                1365

Gly Gly Gly Gly Ser Pro Lys Lys Lys Arg Lys Val Glu Asn Leu
    1370                1375                1380

Tyr Phe Gln Gly His His His His His His
    1385                1390

<210>  2
<211>  70
<212>  DNA
<213>  Artificial Sequence

<220>
<223>  I-SceI LUC Guide it

<400>  2
tgcggcctct aatacgactc actataggga ttacaatgat agggataacg ttttagagct      60

agaaatagca                                                              70

<210>  3
<211>  70
<212>  DNA
<213>  Artificial Sequence

<220>
<223>  GFP-Wx-LUC Guide it

<400>  3
tgcggcctct aatacgactc actatagggc cttataagca catatcgcag ttttagagct      60

agaaatagca                                                            70


<210>  4
<211>  76
<212>  DNA
<213>  Artificial Sequence

<220>
<223>  Guide-it scaffold template

<400>  4
gcaccgactc ggtgccactt tttcaagttg ataacggact agccttattt taacttgcta      60

tttctagctc taaaac                                                     76


<210>  5
<211>  11588
<212>  DNA
<213>  Artificial Sequence

<220>
<223>  pZK-L-I-SceI-UC

<400>  5
agtactttga tccaacccct ccgctgctat agtgcagtcg gcttctgacg ttcagtgcag      60

ccgtcttctg aaaacgacat gtcgcacaag tcctaagtta cgcgacaggc tgccgccctg     120

cccttttcct ggcgttttct tgtcgcgtgt tttagtcgca taaagtagaa tacttgcgac     180

tagaaccgga gacattacgc catgaacaag agcgccgccg ctggcctgct gggctatgcc     240

cgcgtcagca ccgacgacca ggacttgacc aaccaacggg ccgaactgca cgcggccggc     300

tgcaccaagc tgttttccga gaagatcacc ggcaccaggc gcgaccgccc ggagctggcc     360

aggatgcttg accacctacg ccctggcgac gttgtgacag tgaccaggct agaccgcctg     420

gcccgcagca cccgcgacct actggacatt gccgagcgca tccaggaggc cggcgcgggc     480

ctgcgtagcc tggcagagcc gtgggccgac accaccacgc cggccggccg catggtgttg     540

accgtgttcg ccggcattgc cgagttcgag cgttccctaa tcatcgaccg cacccggagc     600

gggcgcgagg ccgccaaggc ccgaggcgtg aagtttggcc cccgccctac cctcaccccg     660

gcacagatcg cgcacgcccg cgagctgatc gaccaggaag gccgcaccgt gaaagaggcg     720

gctgcactgc ttggcgtgca tcgctcgacc ctgtaccgcg cacttgagcg cagcgaggaa     780

gtgacgccca ccgaggccag gcggcgcggt gccttccgtg aggacgcatt gaccgaggcc     840

gacgccctgg cggccgccga gaatgaacgc caagaggaac aagcatgaaa ccgcaccagg     900

acggccagga cgaaccgttt ttcattaccg aagagatcga ggcggagatg atcgcggccg     960

ggtacgtgtt cgagccgccc gcgcacgtct caaccgtgcg gctgcatgaa atcctggccg 1020

gtttgtctga tgccaagctg gcggcctggc cggccagctt ggccgctgaa gaaaccgagc 1080

gccgccgtct aaaaaggtga tgtgtatttg agtaaaacag cttgcgtcat gcggtcgctg 1140

cgtatatgat gcgatgagta aataaacaaa tacgcaaggg gaacgcatga aggttatcgc 1200

tgtacttaac cagaaaggcg ggtcaggcaa gacgaccatc gcaacccatc tagcccgcgc 1260

cctgcaactc gccggggccg atgttctgtt agtcgattcc gatccccagg gcagtgcccg 1320

cgattgggcg gccgtgcggg aagatcaacc gctaaccgtt gtcggcatcg accgcccgac 1380

gattgaccgc gacgtgaagg ccatcggccg gcgcgacttc gtagtgatcg acggagcgcc 1440

ccaggcggcg gacttggctg tgtccgcgat caaggcagcc gacttcgtgc tgattccggt 1500

gcagccaagc ccttacgaca tatgggccac cgccgacctg gtggagctgg ttaagcagcg 1560

cattgaggtc acggatggaa ggctacaagc ggcctttgtc gtgtcgcggg cgatcaaagg 1620

cacgcgcatc ggcggtgagg ttgccgaggc gctggccggg tacgagctgc ccattcttga 1680

gtcccgtatc acgcagcgcg tgagctaccc aggcactgcc gccgccggca caaccgttct 1740

tgaatcagaa cccgagggcg acgctgcccg cgaggtccag gcgctggccg ctgaaattaa 1800

atcaaaactc atttgagtta atgaggtaaa gagaaaatga gcaaaagcac aaacacgcta 1860

agtgccggcc gtccgagcgc acgcagcagc aaggctgcaa cgttggccag cctggcagac 1920

acgccagcca tgaagcgggt caactttcag ttgccggcgg aggatcacac caagctgaag 1980

atgtacgcgg tacgccaagg caagaccatt accgagctgc tatctgaata catcgcgcag 2040

ctaccagagt aaatgagcaa atgaataaat gagtagatga attttagcgg ctaaaggagg 2100

cggcatggaa aatcaagaac aaccaggcac cgacgccgtg gaatgcccca tgtgtggagg 2160

aacgggcggt tggccaggcg taagcggctg ggttgtctgc cggccctgca atggcactgg 2220

aacccccaag cccgaggaat cggcgtgacg gtcgcaaacc atccggcccg gtacaaatcg 2280

gcgcggcgct gggtgatgac ctggtggaga agttgaaggc cgcgcaggcc gcccagcggc 2340

aacgcatcga ggcagaagca cgccccggtg aatcgtggca agcggccgct gatcgaatcc 2400

gcaaagaatc ccggcaaccg ccggcagccg gtgcgccgtc gattaggaag ccgcccaagg 2460

gcgacgagca accagatttt ttcgttccga tgctctatga cgtgggcacc cgcgatagtc 2520

gcagcatcat ggacgtggcc gttttccgtc tgtcgaagcg tgaccgacga gctggcgagg 2580

tgatccgcta cgagcttcca gacgggcacg tagaggtttc cgcagggccg gccggcatgg 2640

ccagtgtgtg ggattacgac ctggtactga tggcggtttc ccatctaacc gaatccatga 2700

accgataccg ggaagggaag ggagacaagc ccggccgcgt gttccgtcca cacgttgcgg 2760

acgtactcaa gttctgccgg cgagccgatg gcggaaagca gaaagacgac ctggtagaaa 2820

cctgcattcg gttaaacacc acgcacgttg ccatgcagcg tacgaagaag gccaagaacg 2880

gccgcctggt gacggtatcc gagggtgaag ccttgattag ccgctacaag atcgtaaaga 2940

gcgaaaccgg gcggccggag tacatcgaga tcgagctagc tgattggatg taccgcgaga 3000

tcacagaagg caagaacccg gacgtgctga cggttcaccc cgattacttt ttgatcgatc 3060

ccggcatcgg ccgttttctc taccgcctgg cacgccgcgc cgcaggcaag gcagaagcca 3120

gatggttgtt caagacgatc tacgaacgca gtggcagcgc cggagagttc aagaagttct 3180

gtttcaccgt gcgcaagctg atcgggtcaa atgacctgcc ggagtacgat ttgaaggagg 3240

aggcggggca ggctggcccg atcctagtca tgcgctaccg caacctgatc gagggcgaag 3300

catccgccgg ttcctaatgt acggagcaga tgctagggca aattgcccta gcaggggaaa 3360

aaggtcgaaa aggtctcttt cctgtggata gcacgtacat tgggaaccca aagccgtaca 3420

ttgggaaccg gaacccgtac attgggaacc caaagccgta cattgggaac cggtcacaca 3480

tgtaagtgac tgatataaaa gagaaaaaag gcgatttttc cgcctaaaac tctttaaaac 3540

ttattaaaac tcttaaaacc cgcctggcct gtgcataact gtctggccag cgcacagccg 3600

aagagctgca aaaagcgcct acccttcggt cgctgcgctc cctacgcccc gccgcttcgc 3660

gtcggcctat cgcggccgct ggccgctcaa aaatggctgg cctacggcca ggcaatctac 3720

cagggcgcgg acaagccgcg ccgtcgccac tcgaccgccg gcgcccacat caaggcaccc 3780

tgcctcgcgc gtttcggtga tgacggtgaa aacctctgac acatgcagct cccggagacg 3840

gtcacagctt gtctgtaagc ggatgccggg agcagacaag cccgtcaggg cgcgtcagcg 3900

ggtgttggcg ggtgtcgggg cgcagccatg acccagtcac gtagcgatag cggagtgtat 3960

actggcttaa ctatgcggca tcagagcaga ttgtactgag agtgcaccat atgcggtgtg 4020

aaataccgca cagatgcgta aggagaaaat accgcatcag gcgctcttcc gcttcctcgc 4080

tcactgactc gctgcgctcg gtcgttcggc tgcggcgagc ggtatcagct cactcaaagg 4140

cggtaatacg gttatccaca gaatcagggg ataacgcagg aaagaacatg tgagcaaaag 4200

gccagcaaaa ggccaggaac cgtaaaaagg ccgcgttgct ggcgtttttc cataggctcc 4260

gcccccctga cgagcatcac aaaaatcgac gctcaagtca gaggtggcga aacccgacag 4320

gactataaag ataccaggcg tttccccctg gaagctccct cgtgcgctct cctgttccga 4380

ccctgccgct taccggatac ctgtccgcct ttctcccttc gggaagcgtg gcgctttctc 4440

atagctcacg ctgtaggtat ctcagttcgg tgtaggtcgt tcgctccaag ctgggctgtg 4500

tgcacgaacc ccccgttcag cccgaccgct gcgccttatc cggtaactat cgtcttgagt 4560

ccaacccggt aagacacgac ttatcgccac tggcagcagc cactggtaac aggattagca 4620

gagcgaggta tgtaggcggt gctacagagt tcttgaagtg gtggcctaac tacggctaca 4680

ctagaaggac agtatttggt atctgcgctc tgctgaagcc agttaccttc ggaaaaagag 4740

ttggtagctc ttgatccggc aaacaaacca ccgctggtag cggtggtttt tttgtttgca 4800

agcagcagat tacgcgcaga aaaaaaggat ctcaagaaga tcctttgatc ttttctacgg 4860

ggtctgacgc tcagtggaac gaaaactcac gttaagggat tttggtcatg catgatatat 4920

ctcccaattt gtgtagggct tattatgcac gcttaaaaat aataaaagca gacttgacct 4980

gatagtttgg ctgtgagcaa ttatgtgctt agtgcatcta atcgcttgag ttaacgccgg 5040

cgaagcggcg tcggcttgaa cgaatttcta gctagacatt atttgccgac taccttggtg 5100

atctcgcctt tcacgtagtg gacaaattct tccaactgat ctgcgcgcga ggccaagcga 5160

tcttcttctt gtccaagata agcctgtcta gcttcaagta tgacgggctg atactgggcc 5220

ggcaggcgct ccattgccca gtcggcagcg acatccttcg gcgcgatttt gccggttact 5280

gcgctgtacc aaatgcggga caacgtaagc actacatttc gctcatcgcc agcccagtcg 5340

ggcggcgagt tccatagcgt taaggtttca tttagcgcct caaatagatc ctgttcagga 5400

accggatcaa agagttcctc cgccgctgga cctaccaagg caacgctatg ttctcttgct 5460

tttgtcagca agatagccag atcaatgtcg atcgtggctg gctcgaagat acctgcaaga 5520

atgtcattgc gctgccattc tccaaattgc agttcgcgct tagctggata acgccacgga 5580

atgatgtcgt cgtgcacaac aatggtgact tctacagcgc ggagaatctc gctctctcca 5640

ggggaagccg aagtttccaa aaggtcgttg atcaaagctc gccgcgttgt ttcatcaagc 5700

cttacggtca ccgtaaccag caaatcaata tcactgtgtg gcttcaggcc gccatccact 5760

gcggagccgt acaaatgtac ggccagcaac gtcggttcga gatggcgctc gatgacgcca 5820

actacctctg atagttgagt cgatacttcg gcgatcaccg cttcccccat gatgtttaac 5880

tttgttttag ggcgactgcc ctgctgcgta acatcgttgc tgctccataa catcaaacat 5940

cgacccacgg cgtaacgcgc ttgctgcttg gatgcccgag gcatagactg taccccaaaa 6000

aaacatgtca taacaagaag ccatgaaaac cgccactgcg ccgttaccac cgctgcgttc 6060

ggtcaaggtt ctggaccagt tgcgtgacgg cagttacgct acttgcatta cagcttacga 6120

accgaacgag gcttatgtcc actgggttcg tgcccgaatt gatcacaggc agcaacgctc 6180

tgtcatcgtt acaatcaaca tgctaccctc cgcgagatca tccgtgtttc aaacccggca 6240

gcttagttgc cgttcttccg aatagcatcg gtaacatgag caaagtctgc cgccttacaa 6300

cggctctccc gctgacgccg tcccggactg atgggctgcc tgtatcgagt ggtgattttg 6360

tgccgagctg ccggtcgggg agctgttggc tggctggtgg caggatatat tgtggtgtaa 6420

acaaattgac gcttagacaa cttaataaca cattgcggac gtttttaatg tactgaatta 6480

acgccgaatt gctctagcca atacgcaaac cgcctctccc cgcgcgttgg ccgattcatt 6540

aatgcagctg gcacgacagg tttcccgact ggaaagcggg cagtgagcgc aacgcaatta 6600

atgtgagtta gctcactcat taggcacccc aggctttaca ctttatgctt ccggctcgta 6660

tgttgtgtgg aattgtgagc ggataacaat ttcacacagg aaacagctat gaccatgatt 6720

acgaatttta ccaggtcctg ctgagcctcg acatgttgtc gcaaaattcg ccctggaccc 6780

gcccaacgat ttgtcgtcac tgtcaaggtt tgacctgcac ttcatttggg gcccacatac 6840

accaaaaaaa tgctgcataa ttctcggggc agcaagtcgg ttacccggcc gccgtgctgg 6900

accgggttga atggtgcccg taactttcgg tagagcggac ggccaatact caacttcaag 6960

gaatctcacc catgcgcgcc ggcggggaac cggagttccc ttcagtgagc gttattagtt 7020

cgccgctcgg tgtgtcgtag atactagccc ctggggcact tttgaaattt gaataagatt 7080

tatgtaatca gtcttttagg tttgaccggt tctgccgctt tttttaaaat tggatttgta 7140

ataataaaac gcaattgttt gttattgtgg cgctctatca tagatgtcgc tataaaccta 7200

ttcagcacaa tatattgttt tcattttaat attgtacata taagtagtag ggtacaatca 7260

gtaaattgaa cggagaatat tattcataaa aatacgatag taacgggtga tatattcatt 7320

agaatgaacc gaaaccggcg gtaaggatct gagctacaca tgctcaggtt ttttacaacg 7380

tgcacaacag aattgaaagc aaatatcatg cgatcatagg cgtctcgcat atctcattaa 7440

agcaggactc tagtcgctca gaagaactcg tcaagaaggc gatagaaggc gatgcgctgc 7500

gaatcgggag cggcgatacc gtaaagcacg aggaagcggt cagcccattc gccgccaagc 7560

tcttcagcaa tatcacgggt agccaacgct atgtcctgat agcggtccgc cacacccagc 7620

cggccacagt cgatgaatcc agaaaagcgg ccattttcca ccatgatatt cggcaagcag 7680

gcatcgccat gggtcacgac gagatcctcg ccgtcgggca tgcgcgcctt gagcctggcg 7740

aacagttcgg ctggcgcgag cccctgatgc tcttcgtcca gatcatcctg atcgacaaga 7800

ccggcttcca tccgagtacg tgctcgctcg atgcgatgtt tcgcttggtg gtcgaatggg 7860

caggtagccg gatcaagcgt atgcagccgc cgcattgcat cagccatgat ggatactttc 7920

tcggcaggag caaggtgaga tgacaggaga tcctgccccg gcacttcgcc caatagcagc 7980

cagtcccttc ccgcttcagt gacaacgtcg agcacagctg cgcaaggaac gcccgtcgtg 8040

gccagccacg atagccgcgc tgcctcgtcc tgcagttcat tcagggcacc ggacaggtcg 8100

gtcttgacaa aaagaaccgg gcgcccctgc gctgacagcc ggaacacggc ggcatcagag 8160

cagccgattg tctgttgtgc ccagtcatag ccgaatagcc tctccaccca agcggccgga 8220

gaacctgcgt gcaatccatc ttgttcaatc atggttagat ctggattgag agtgaatatg 8280

agactctaat tggataccga ggggaattta tggaacgtca gtggagcatt tttgacaaga 8340

aatatttgct agctgatagt gaccttaggc gacttttgaa cgcgcaataa tggtttctga 8400

cgtatgtgct tagctcatta aactccagaa acccgcggct gagtggctcc ttcaacgttg 8460

cggttctgtc agttccaaac gtaaaacggc ttgtcccgcg tcatcggcgg gggtcataac 8520

gtgactccct taattctccg ctcatgatct tgatcccctg cgccatcaga tccttggcgg 8580

caagaaagcc atccagttta ctttgcaggg cttcccaacc ttaccagagg gcgccccagc 8640

tggcaattcc ggttcgcttg ctgtccataa aaccgcccag tctagctatc gccatgtaag 8700

cccactgcaa gctacctgct ttctctttgc gcttgcgttt tcccttgtcc agatagccca 8760

gtagctgaca ttcatccggg gtcagcaccg tttctgcgga ctggctttct acgtgttccg 8820

cttcctttag cagcccttgc gccctgagtg cttgcggcag cgtgaagctt ttacgtagtc 8880

gacaggcctg gcgcgccatt ccattgccca gctatctgtc acttcatcga aaggacagta 8940

gaaaaggaag gtggctccta caaatgccat cattgcgata aaggaaaggc tatcgttcaa 9000

gatgcctcta ccgacagtgg tcccaaagat ggacccccac ccacgaggaa catcgtggaa 9060

aaagaagacg ttccaaccac gtcttcaaag caagtggatt gatgtgatat ctccactgac 9120

gtaagggatg acgcacaatc ccactatcct tcgcaagacc cttcctctat ataaggaagt 9180

tcatttcatt tggagaggac aggcttcttg agatccttca acaattacca acaacaacaa 9240

acaacaaaca acattacaat tactatttac aattacaatg atagggataa cagggtaatg 9300

agagagagaa gaacgtggtg tacggccccg agcccaagca ccctctgggc aacttcaccg 9360

ccggcgagat gctgtacaac gctctgcaca agcactccca catcccccag gccatcctgg 9420

acgtgatggg caacgagtcc ctttcctacc aggagttctt cgacactact gtgaagctgg 9480

gccagagcct ccagaactgt ggctacaaga tgaacgatgt cgtgtcgatc tgtgcagaga 9540

acaacaagag attcttcatc cccatcatct ccgcctggta catcggcatg gtggtggccc 9600

ctgtgaacga ggactatatc ccagacgagc tgtgtaaagt gaccggcatc tccaagccga 9660

tcctggtctt caccactagg aagatcctgc ctaaggtttt ggaggttaaa gacagaacca 9720

actacataaa gaggatcatc atactggact ctgaagagaa cctgctgggc tgcgagagcc 9780

tgcacaactt catgtccagg tactccgaca acaacctcca aacattcaag cctctgcact 9840

acgaccctgt ggaccaggta gccgccatcc tgtgctcctc cggcacaacc ggcctgccta 9900

aaggcgtgat gcagacccac aggaacatct gtgtgagact cacacacgca tctgacccca 9960

gagtgggtac acaactcatc cccggcgtat ccgtgctggc ctacctgcca ttcttccacg 10020

ccttcggctt cagtatcaac ctgggctatt tcatggtggg cctgagagtg gtgatgctcc 10080

gaaggtttaa ccaggaggtg ttcctgaagg ccatccagga ctacgaggtg aggagcgtga 10140

tcaacgttcc ctccacaatc ctgttcctgt ccaagagccc tctggtggac aagtacgacc 10200

tatccaccct ggcggagctg tgctgtggag ccgctcctct ggcgaaggag gtggccgaga 10260

tcgccgtgaa gaggctgaac ctgccaggga tacggtgtgg ctacggtcta acagagtcta 10320

cctccgccaa catccatact ctgcacaacg agttcaagtc cggctccctg ggcaaggtga 10380

caccttacat ggccgccaag atcatcgaca ggaacaccgg cgaggccctg ggtccaaacc 10440

aggtgggcga gctgtgcatc tggggaccta tggtaacaaa aggctatgtg aacaacccac 10500

aggctactaa ggaggccatc gacgacgacg gctggctgca ctctggcgac ttcggctact 10560

acgacgagga cgagtatttc tacatcgtgg accggtacaa ggagctgatc aaatacaagg 10620

gctatcaggt cgcccctgtg gagctggagg agatcctcct tcagcaccca ggcatcaggg 10680

acgtggccgt cgtgggtatc cctgacatcg aggccggcga gctgccagcc ggcttcgtgg 10740

tgaagcagcc cggcgcccaa ctcaccgcta aggaggtgta cgacttcctg gcccagaggg 10800

tgtctcactc caagtacctg aggggcggcg taaggttcgt ggactctatc cccaggaacg 10860

tgacaggcaa gattagtcga aaagagctga gggaggccct gatggagaag gcttctaagc 10920

tgtaatctag agcggccgcc cggctgcaga tcgttcaaac atttggcaat aaagtttctt 10980

aagattgaat cctgttgccg gtcttgcgat gattatcata taatttctgt tgaattacgt 11040

taagcatgta ataattaaca tgtaatgcat gacgttattt atgagatggg tttttatgat 11100

tagagtcccg caattataca tttaatacgc gatagaaaac aaaatatagc gcgcaaacta 11160

ggataaatta tcgcgcgcgg tgtcatctat gttactagat ccgatgataa gctgtcaaac 11220

atgagaattc agcttggcac tggccgtcgt tttacaacgt cgtgactggg aaaaccctgg 11280

cgttacccaa cttaatcgcc ttgcagcaca tccccctttc gccagctggc gtaatagcga 11340

agaggcccgc accgatcgcc cttcccaaca gttgcgcagc ctgaatggcg aatgagcttg 11400

agcttggatc agattgtcgt ttcccgcctt cagtttaaac tatcagtgtt tgacaggata 11460

tattggcggg taaacctaag agaaaagagc gtttattaga ataacggata tttaaaaggg 11520

cgtgaaaagg tttatccgtt cgtccatttg tatgtgcatg ccaaccacag ggttcccctc 11580

gggatcaa 11588

<210> 6
<211> 85
<212> DNA
<213> Artificial Sequence

<220>
<223> Original partial sequence 1 of L-(I-SceI)-UC

<400> 6
ttaccaacaa caacaaacaa caaacaacat tacaattact atttacaatt acaatgatag 60

ggataacagg gtaatgagag agaga 85

<210> 7
<211> 84
<212> DNA
<213> Artificial Sequence

<220>
<223> Mutated partial sequence 1 of L-(I-SceI)-UC

<400> 7
ttaccaacaa caacaaacaa caaacaacat tacaattact atttacaatt acaatgatag 60

ggataaaagg taatgagaga gaga 84

<210> 8
<211> 48
<212> DNA
<213> Artificial Sequence

<220>
<223> Original partial sequence 2 of L-(I-SceI)-UC

<400> 8
actatttaca attacaatga tagggataac agggtaatga gagagaga 48

<210> 9
<211> 48
<212> DNA
<213> Artificial Sequence

<220>
<223> Partially complementary original sequence 2 of L-(I-SceI)-UC

<400> 9

tgataaatgt taatgttact atccctattg tcccattact ctctctct 48

<210> 10
<211> 20
<212> DNA
<213> Artificial Sequence

<220>
<223> sgRNA target sequence

<400> 10
attacaatga tagggataac 20

<210> 11
<211> 2413
<212> DNA
<213> Artificial Sequence

<220>
<223> sGFP-wTALEN-ELUC

<400> 11
atggtgagca agggcgagga gctgttcacc ggggtggtgc ccatcctggt cgagctggac 60

ggcgacgtga acggccacaa gttcagcgtg tccggcgagg gcgagggcga tgccacctac 120

ggcaagctga ccctgaagtt catctgcacc accggcaagc tgcccgtgcc ctggcccacc 180

ctcgtgacca ccttcaccta cggcgtgcag tgcttcagcc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actcacggca tggacgagct gtacaagtct 720

agaatggtcc ttataagcac atatcgcatg gtaccatata tgtttgagtt ttagcgacga 780

attcatggag agagagaaga acgtggtgta cggccccgag cccaagcacc ctctgggcaa 840

cttcaccgcc ggcgagatgc tgtacaacgc tctgcacaag cactcccaca tcccccaggc 900

catcctggac gtgatgggca acgagtccct ttcctaccag gagttcttcg acactactgt 960

gaagctgggc cagagcctcc agaactgtgg ctacaagatg aacgatgtcg tgtcgatctg 1020

tgcagagaac aacaagagat tcttcatccc catcatctcc gcctggtaca tcggcatggt 1080

ggtggcccct gtgaacgagg actatatccc agacgagctg tgtaaagtga ccggcatctc 1140

caagccgatc ctggtcttca ccactaggaa gatcctgcct aaggttttgg aggttaaaga 1200

cagaaccaac tacataaaga ggatcatcat actggactct gaagagaacc tgctgggctg 1260

cgagagcctg cacaacttca tgtccaggta ctccgacaac aacctccaaa cattcaagcc 1320

tctgcactac gaccctgtgg accaggtagc cgccatcctg tgctcctccg gcacaaccgg 1380

cctgcctaaa ggcgtgatgc agacccacag gaacatctgt gtgagactca cacacgcatc 1440

tgaccccaga gtgggtacac aactcatccc cggcgtatcc gtgctggcct acctgccatt 1500

cttccacgcc ttcggcttca gtatcaacct gggctatttc atggtgggcc tgagagtggt 1560

gatgctccga aggtttaacc aggaggtgtt cctgaaggcc atccaggact acgaggtgag 1620

gagcgtgatc aacgttccct ccacaatcct gttcctgtcc aagagccctc tggtggacaa 1680

gtacgaccta tccaccctgg cggagctgtg ctgtggagcc gctcctctgg cgaaggaggt 1740

ggccgagatc gccgtgaaga ggctgaacct gccagggata cggtgtggct acggtctaac 1800

agagtctacc tccgccaaca tccatactct gcacaacgag ttcaagtccg gctccctggg 1860

caaggtgaca ccttacatgg ccgccaagat catcgacagg aacaccggcg aggccctggg 1920

tccaaaccag gtgggcgagc tgtgcatctg ggacctatgg gtaacaaaag gctatgtgaa 1980

caacccacag gctactaagg aggccatcga cgacgacggc tggctgcact ctggcgactt 2040

cggctactac gacgaggacg agtatttcta catcgtggac cggtacaagg agctgatcaa 2100

atacaagggc tatcaggtcg cccctgtgga gctggaggag atcctccttc agcacccagg 2160

catcagggac gtggccgtcg tgggtatccc tgacatcgag gccggcgagc tgccagccgg 2220

cttcgtggtg aagcagcccg gcgcccaact caccgctaag gaggtgtacg acttcctggc 2280

ccagagggtg tctcactcca agtacctgag gggcggcgta aggttcgtgg actctatccc 2340

caggaacgtg acaggcaaga ttagtcgaaa agagctgagg gaggccctga tggagaaggc 2400

ttctaagctg taa 2413

<210> 12
<211> 55
<212> DNA
<213> Artificial Sequence

<220>
<223> waxy TALEN target sequnece

<400> 12

atggtcctta taagcacata tcgcatggta ccatatatgt ttgagtttta gcgac 55